# EUROPÄISCHE PATENTSCHRIFT

(11) **EP 1 131 008 B1**
(45) Veröffentlichungstag und Bekanntmachung des Hinweises auf die Patenterteilung: **10.12.2003**
(21) Anmeldenummer: 98952492.1
(22) Anmeldetag: 17.11.1998
(51) Int. Cl.: A61B 17/72

(54) **MARKRAUMNAGEL ZUR OPERATIVEN BEHANDLUNG VON UNTERARMFRAKTUREN**
INTRAMEDULLARY NAIL FOR THE OPERATIVE TREATMENT OF FRACTURES OF THE LOWER ARM
CLOU D'OSTEOSYNTHESE INTRAMEDULLAIRE POUR LE TRAITEMENT OPERATOIRE DE FRACTURES DE L'AVANT BRAS

(43) Veröffentlichungstag der Anmeldung: 12.09.2001
(73) Patentinhaber: SYNTHES AG Chur, 7002 Chur (CH)
(72) Erfinder: FRIGG, Robert, CH-2544 Bettlach (CH)
(74) Vertreter: Lusuardi, Werther Giovanni, Dr.
(86) Internationale Anmeldenummer: CH9800494
(87) Internationale Veröffentlichungsnummer: WO00028906

(56) Entgegenhaltungen:
- EP-A- 0 551 846
- WO-A-97/10767
- WO-A-98/24380
- DE-A- 4 143 362
- FR-A- 2 237 609
- FR-A- 2 656 212
- US-A- 4 457 301
- US-A- 5 009 664
- US-A- 5 116 335
- US-A- 5 814 047

## Beschreibung

Die Erfindung bezieht sich auf einen Markraumnagel zur operativen Frakturbehandlung von dünnen Röhrenknochen am menschlichen Körper gemäss dem Oberbegriff des Patentanspruchs 1.

Zur operativen Frakturbehandlung von Unterarmfrakturen werden üblicherweise Platten und Schrauben eingesetzt. Im Gegensatz zur Marknagelung bei anderen Röhrenknochen (Tibia, Femur und Humerus) hat sich die Frakturbehandlung an der Ulna oder am Radius mit Marknägeln bisher nicht durchgesetzt. In einzelnen Fällen wurden dünne Marknägel verwendet, welche paarweise eingesetzt wurden. Damit lässt sich eine gewisse Schienung der Knochens erreichen. Allerdings kann die Rotation der Knochenfragmente nicht verhindert werden, da die Nägel nicht miteinander verbunden sind. Zusätzlich sind die Marknägel nur durch Verspannung im Markraum fixiert, so dass das Herauswandern von dünnen Nägeln problematisch werden kann. Versuche mit herkömmlichen verriegelungsnägeln scheiterten daran, dass der kleine Nagelquerschnitt nur sehr kleine Querbohrungen zulässt, welche auch mit einem Bildverstärker nur schwer zu orten sind. Auch sind die verwendbaren Verriegelungsbolzen zu klein, um die auftretenden Kräfte aufzunehmen.

Aus der US 4,805,607 ENGELHARDT ist bereits ein Markraumnagel bekannt, welcher einen nicht runden, vorzugsweise dreikantigen Querschnitt mit konkaven Seitenflächen aufweist. Diese Gestalt des Nagelkörpers gestattet eine Implantation des Markraumnagels im Markraum eines Knochens ohne vorgängiges Aufbohren des Markraumkanals mittels eines Räumwerkzeuges. Der dreikantige Nagelkörper gestattet einen kortikalen Kontakt entlang drei sehr dünner Metallkanten. Diese lokale Gestaltung des kortikalen Kontaktes führt zu einer stark verminderten Gefahr der Rotation des Markraumnagels im Markraum.

Ein weiterer Marknagel zur operativen Behandlung von Unterarmfrakturen mit einem sternförmigen Nagelquerschnitt wird von der Firma Applied Osteo Systems, Inc., Walnut Creek, California unter dem Namen Trueflex Nail auf dem Markt vertrieben. Neben der Schienung des Knochens sollte der stemförmige Nagelquerschnitt ein Rotieren der Hauptfragmente verhindern. Das ist jedoch nur möglich, wenn der Nagel soweit wie möglich in den Markraum vorgeschlagen wird, damit sich die Nagelspitze in der gelenksnaben Spongiosa verankem kann. Das hat jedoch zur Folge, dass die Frakturzone distrahiert wird, was zu einer Störung der Frakturheilung führt.

Ein intramedullärer Marknagel gemäß dem Oberbegriff von Anspruch 1 mit einem Kern und drei relativ zur Längsachse des Kerns verschiebbaren flexiblen Stäben ist aus der US 5,116,335 HANNON bekannt. Der Kern dieses bekannten Marknagels umfasst drei Nuten, weiche auf dem Umfang des Kerns in Winkeln von 120° zueinander angeordnet sind und sich über die gesamte Länge des Kerns erstrecken. Auf einem Längsabschnitt des Kerns sind die Nuten im Querschnitt so ausgebildet, dass sie sich gegen die äussere Mantelfläche des Kerns hin verjüngen und somit die Stäbe gegen die Zentralachse des Kerns festhalten. Am proximalen und am distalen Ende des Marknagels verjüngen sich die Querschnitte der Nuten nicht gegen die äussere Mantelfläche des Kerns, so dass die Stäbe quer zur Zentralachse des Kerns ausgebogen und im Knochen verankert werden können.

Nachteilig bei diesen bekannten Marknägeln ist die Gefahr der Rotation des Marknagels im Markraum. Diese Gefahr wird durch die Ausgestaltung mit einem sternförmigen Querschnitt, was einen kortikalen Kontakt der Kanten ermöglicht, nicht ausreichend beseitigt.

Hier will die Erfindung Abhilfe schaffen. Der Erfindung liegt die Aufgabe zugrunde, einen Markraumnagel für Unterarmfrakturen derart zu gestalten, dass eine Rotation des Marknagels im Markraum verhindert wird und zudem der Marknagel fixierbar ist.

Die Erfindung löst die gestellte Aufgabe mit einem Markraumnagel zur operativen Frakturbehandlung von dünnen Röhrenknochen am menschlichen Körper, welcher die Merkmale des Anspruchs 1 aufweist.

Die durch die Erfindung erreichten Vorteile sind im wesentlichen darin zu sehen, dass dank des erfindungsgemässen Marknagels
- eine kontrollierte und sichere intramedulläre Frakturversorgung eines dünnen Röhrenknochens, beispielsweise Radius, Ulna oder kindliche Röhrenknochen möglich ist;
- ein flexibles Implantat, welches die Wahl von verschiedenen Eintrittsorten zulässt (retrograd, antegrad) verfügbar ist; und
- durch Zurückziehen der Verriegelungsdrähte eine Überdistraktion der Fraktur korrigierbar ist.

Der erfindungsgemässe Marknagel umfasst einen Nagelkörper mit parallel und exzentrisch zur Längsachse angebrachten Nuten, welche zur Aufnahme von parallel zum Marknagel verlaufenden Verriegelungsdrähten dienen, zwei Verriegelungsdrähte und eine Verriegelungsschraube. Die Verriegelungsdrähte sind in den Nuten drehbar und in Längsrichtung des Marknagels verschiebbar.

Eine Sicherung gegen Rotation und axiale Verschiebung des Marknagels wird durch Einsetzen einer quer zum Marknagel verlaufenden Verriegelungsschraube erreicht. Zu diesem Zweck ist am Nagelkörper im Bereich des oberen Endes eine quer zur Längsachse verlaufende Durchgangsbohrung angebracht.

Im Bereich der Durchgangsbohrung weisen die Nuten eine Aussparung auf, wodurch die Verriegelungsdrähte im Bereich der Durchgangsbohrung um die Verriegelungsschraube auskröpfbar sind. Durch die ausgekröpften, jedoch an die Verriegelungsschraube anliegenden Verriegelungsdrähte, wird der Marknagel wegen der Verschraubung der Verriegelungsdrähte im Knochen zusätzlich axial gesichert. Zudem wird eine zusätzlich zur Verriegelungsschraube wirkende einfache und dennoch wirksame Verriegelung der Nagelspitze erreicht und der Nagelquerschnitt kann klein gehalten werden.

Je nach Grösse des Markraumes muss dieser vor dem Einführen des Marknagels nicht mit einem Bohrer vorbereitet werden. Beträgt der Markraumdurchmesser mindestens 6 mm, kann der Marknagel ohne Vorbohrung in den Markraum eingeführt werden. Andernfalls muss der Markraum analog zur Humerus-, Femur- oder Tibianagelung vorgängig aufgebohrt werden.

In einer bevorzugten Ausführungsform des erfindungsgemässen Marknagels sind die Verriegelungsdrähte an ihren bei der Nagelspitze liegenden Enden mit Gewinden versehen. Zudem sind die Nuten im Bereich der Nagelspitze mit dazu korrespondierenden Innengewinden ausgestattet, so dass beim Einführen des Marknagels in den Markraum die Verriegelungsdrähte durch Einschrauben derselben in die Innengewinde diese am Marknagel befestigt sind. Sobald das proximale Nagelende mit dem Knochen bündig ist, werden die beiden Verriegelungsdrähte nacheinander von Hand oder mit einer Bohrmaschine in Richtung des Knochenendes in den Knochen eingeschraubt, wobei vorgängig die Gewinde an den Verriegelungsdrähten aus den Innengewinden in den Nuten herausgedreht werden. Da die Metaphyse in der distalen Ulna oder bei der Nagelung des Radius von distal nach proximal, im proximalen Radius sehr hart ist, wird eine gute Verankerung der Verriegelungsdrähte im Knochen erreicht.

Bei älteren Patienten und bei fortgeschrittener Osteoporose kann die fehlende Knochensubstanz an diesen Orten durch biodegradierbaren Zement ersetzt werden, so dass auch dort die Verriegelungsdrähte in diesem Zement einen guten Halt finden.

Nachdem die Verriegelungsdrähte gut im Knochen verankert sind, kann der Frakturspalt durch Zurückziehen der Verriegelungsdrähte und gleichzeitigem Festhalten des Nagelkörpers geschlossen werden. Nach der letzten Kontrolle der Reposition der Fraktur wird die proximale Verriegelungsschraube über ein Zielgerät oder mittels Röntgenkontrolle gesetzt. Durch das Einführen der Verriegelungsschraube in den Nagelkörper werden die beiden Verriegelungsdrähte an dieser Stelle leicht nach aussen gekröpft, wobei sie axial und rotativ blockiert werden. Nach diesem Vorgang ist die Fraktur stabil versorgt. Der Marknagel ist in der einen Metaphyse durch die beiden Verriegelungsdrähte axial und rotativ gesichert und in der anderen Metaphyse durch die Verriegelungsschraube wiederum axial und rotativ gesichert. Dank dieser Verriegelungstechnik muss der Nagel nicht mit Gewalt in den Knochen eingeschlagen werden, um einen Formschluss zu erreichen. Auch müssen keine kleinen Verriegelungslöcher im Bereich der Nagelspitze durch ein Röntgengerät oder einen Bildverstärker aufgefunden und durch eine Schraube verriegelt werden.

Neben der Behandlung der Unterarmfrakturen (Radius und Ulna) ist diese Art der Marknagelung auch für kindliche Frakturen im Bereich Ober-, Unterschenkel sowie im Oberarm einsetzbar. Je nach Grösse und Ausführungsform des Gewindes an den Spitzen der Verriegelungsdrähte kann die Fraktur auf Rotation und axiale Stauchung gesichert werden und trotzdem ein Wachstum des Röhrenknochens zulassen, da der Nagel auf Zug und daher auf Wachstum nicht blockiert ist.

Weitere vorteilhafte Ausgestaltungen der Erfindung sind in den abhängigen Ansprüchen gekennzeichnet.

vorteilhafterweise verengt sich der Querschnitt der Nuten an deren Öffnungen in der Mantelfläche des Nagelkörpers. Damit lässt sich erreichen, dass die Verriegelungsdrähte quer zur Längsachse nicht aus den Nuten entweichen können. Diese Massnahme führt zu einer stabilen Sicherung gegen Rotation des Marknagels im Markraum.

Der Schaft des Nagelkörpers weist vorteilhafterweise einen abgeflachten Querschnitt auf, wobei aus einer Kreisfläche mit dem Durchmesser D zwei Segmente mit parallelen Sehnen, welche symmetrisch zur Längsachse angeordnet sind und einen Abstand x aufweisen, entfernt sind. Dabei liegt das Verhältnis x/D vorteilhafterweise in einem Bereich von 60% bis 70%.

Damit die Verriegelungsdrähte bei einem minimalen Nagelquerschnitt den grösstmöglichen Abstand einnehmen, sind die Nuten in die peripheren Kreisbogen des Schaftquerschnitts eingelassen. Damit lässt sich erreichen, dass der Marknagel trotz kontrollierter Verriegelung kleine Dimensionen aufweisen kann.

Zur Kupplung an Montage- oder Demontageinstrumente weist der von der Nagelspitze entfernte Endteil des Marknagels vorteilhafterweise einen kreiszylindrischen Querschnitt auf. Ebenfalls denkbar ist ein prismatischer oder zylindrischer Querschnitt.

Die Materialwahl des Nagelkörpers ist je nach gewünschter Stabilität zwischen rostfreiem Stahl, Titan oder bei einer höheren geforderten Flexibilität auch Kunststoff möglich. Auch sind verschiedene Materialkombinationen zwischen dem Material des Nagelkörpers und demjenigen der Verriegelungsdrähte denkbar.

Eine bevorzugte Ausführungsform der Erfindung wird im folgenden anhand der Zeichnungen noch näher erläutert.

Es zeigen:
Fig. 1 eine Ansicht der bevorzugten Ausführungsform des erfindungsgemässen Marknagels;
Fig. 2 einen Längsschnitt den in Fig. 1 dargestellten erfindungsgemässen Marknagel;
Fig. 3 eine Querschnitt des in den Fig. 1 und 2 dargestellten erfindungsgemässen Marknagels; und
Fig. 4 eine Vergrösserung des in Fig. 3 dargestellten Querschnittes des erfindungsgemässen Marknagels.

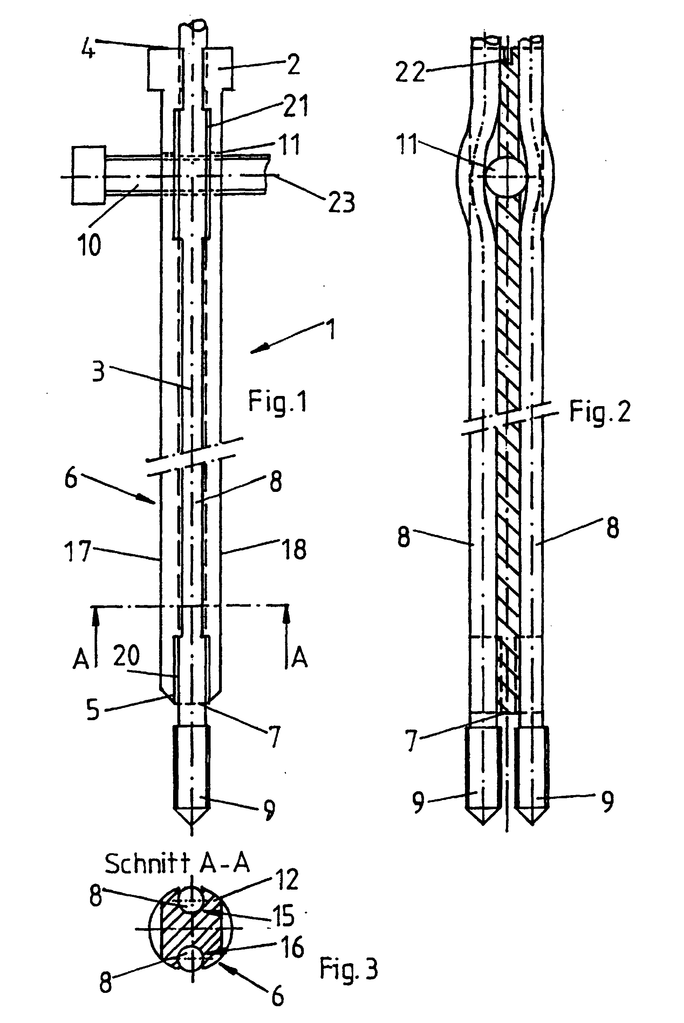

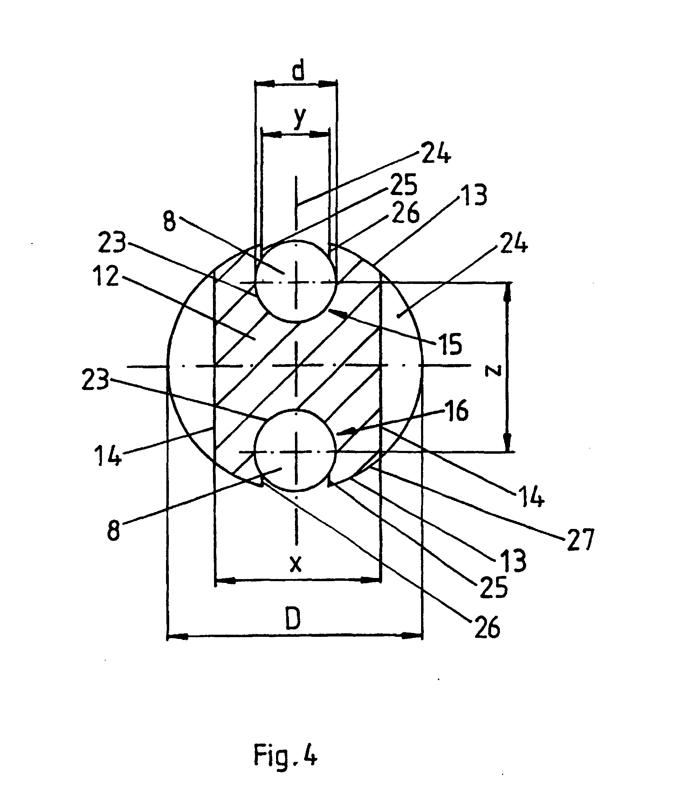

In den Fig. 1, 2 und 3 ist eine bevorzugte Ausführungsform des erfindungsgemässen Markraumnagels bestehend aus einem Nagelkörper 1, einer Verriegelungsschraube 10 und zwei Verriegelungsdrähten 8 dargestellt. Der Nagelkörper 1 mit der Längsachse 3, dem oberen Ende 4 und dem unteren Ende 5 umfasst ein sich am oberen Ende 4 befindendes kreiszylindrisches Endteil 2, welches zur Kupplung an Montage- oder Demontagewerkzeuge dient, einen daran anschliessenden Schaft 6 und eine sich am unteren Ende befindliche Nagelspitze 7 zur Vereinfachung der Implantation. Der Durchmesser D des zylindrischen Endteils 2 beträgt D = 6 mm. Der an das Endteil 2 anschliessende Schaft 6 weist gegenüber dem Endteil 2 einen abgeflachten Querschnitt 12 auf. Wie in Fig. 4 dargestellt sind im Querschnitt 12 aus der Kreisfläche 24 mit dem Zentrum auf der Längsachse 3 und dem Durchmesser D zwei Segmente mit parallelen Sehnen 14, welche symmetrisch zur Längsachse 3 angeordnet sind und einen Abstand x = 4 mm aufweisen, entfernt. An den beiden zwischen den Sehnen 14 verbleibenden peripheren Kreisbogen 13 des Querschnitts 12 sind parallel zur Längsachse 3 diametral gegenüberliegend zwei Nuten 15;16 eingelassen, welche zur Aufnahme der Verriegelungsdrähte 8 dienen. Die symmetrisch zu den peripheren Kreisbogen 14 angeordneten Nuten 15;16 liegen auf einer diametral verlaufenden Symmetrieachse 24 und bestehen aus Bohrungen 23, welche einen Durchmesser d = 2 mm und einen Abstand z = 4 mm zueinander aufweisen. Gegen die peripheren Kreisbogen 13 hin sind die Nuten 15;16 durch parallele, in Richtung des Längsachse 3 verlaufende Seitenflächen 25;26 offen. Die Seitenflächen 25;26 haben senkrecht zur Längsachse 3 einen Abstand y < d, so dass sich die Nuten 15;16 gegen die Mantelfläche 27 des Schaftes 6 verengen, wodurch die Verriegelungsdrähte 8 in Richtung der Längsachse 3 in den Nuten 15;16 verschiebbar sind, jedoch nicht seitlich aus den Nuten 15;16 entweichen können.

Im Bereich des unteren Endes 5 sind die Nuten 15;16 mit Innengewinden 20 versehen, welche mit den Aussengewinden an den Gewindespitzen 9 der Verriegelungsdrähte 8 korrespondieren. Auf diese Weise lassen sich die Verriegelungsdrähte 8 mit den Gewindespitzen 9 beim Einführen des Nagelkörpers 1 in den Markraum in die Nuten 15;16 zurückschrauben, so dass sie sich beim Einführen nicht im Markraum verhaken. Nach der Implantation des Nagelkörpers 1 werden die Verriegelungsdrähte 8 von Hand oder maschinell aus dem Nagelkörper 1 gegen die Nagelspitze 7 hin herausgedreht und in die Spongiosa und/oder Kortikalis des Knochens geschraubt. Da die Verriegelungsdrähte 8 wesentlich länger als der Nagelkörper 1 sind, kann die Eindringtiefe im metaphysären Bereich frei gewählt werden. Nach Abschluss der Nagelung werden die vorstehenden Enden der Verriegelungsdrähte 8 abgetrennt. Bei der Versorgung von porotischen Knochen kann zusätzlich Biozement (z.B. Nioran oder sonstige abbaubare Zemente) verwendet werden. In den diaphysären Bereich eingespritzt, gewährleistet dieser Zement eine gute Verankerung der Verriegelungsdrähte 8, welche aber jeder Zeit durch Herausdrehen entfernt werden können.

Die Verriegelung beim oberen Ende 4 des Nagelkörpers 1 erfolgt über eine Verriegelungsschraube 10, welche in die den Schaft 6 quer zur Längsachse 3 durchdringende Bohrung 11 eingesetzt wird. Damit die Verriegelungsdrähte 8 im Bereich der Bohrung 11 diese nicht verdecken, sind an den Nuten 15;16 in diesem Bereich symmetrisch zur Bohrung 11 Aussparungen 21 angebracht, so dass die Verriegelungsdrähte 8 aus den Nuten 15;16 entweichen können und um die Verriegelungsschraube 10 auskröpfbar sind. Dieses Ausweichen der Verriegelungsdrähte 8 führt zu einer weiteren Verklemmung der Verriegelungsdrähte 8. Dadurch ist die Fraktur nicht nur relativ sondern auch gegen axiale Längenverschiebung gesichert.

## Patentansprüche

1. Markraumnagel zur operativen Frakturbehandlung von dünnen Röhrenknochen am menschlichen Körper, mit
A) einem Nagelkörper (1) mit einer Längsachse (3), einem oberen Ende (4) und einem unteren Ende (5); wobei
B) der Nagelkörper (1) ein sich am oberen Ende (4) befindendes prismatisches oder zylindrisches Endteil (2) mit Mitteln zur Kupplung an Montage- beziehungsweise Demontageinstrumente, einen longitudinalen Schaft (6) und eine sich am unteren Ende (5) befindliche Nagelspitze (7) zur Vereinfachung der Implantation umfasst; und
C) der Nagelkörper (1) parallel und exzentrisch zur Längsachse (3) angeordnete Nuten (15;16) oder Bohrungen zur Aufnahme von Verriegelungsdrähten (8) aufweist, so dass die Verriegelungsdrähte (8) in den Nuten (15;16) oder Bohrungen koaxial zur Längsachse (3) und über die Nagelspitze (7) hinaus verschiebbar sind,
**dadurch gekennzeichnet, dass**
D) der Nagelkörper (1) im Bereich des oberen Endes (4) eine quer zur Längsachse (3) verlaufende Durchgangsbohrung (11) mit einer Zentralachse (23) aufweist, so dass eine Verriegefungsschraube (10) in diese Durchgangsbohrung (11) einführbar ist und dadurch eine Bewegung des Nagelkörpers (1) relativ zur Längsachse (3) sowie eine Rotation des Nagelkörpers (1) um die Längsachse (3) verhindert, und
E) die Nuten (15;16) im Bereich der Durchgangsbohrung (11) eine Aussparung (21) aufweisen, so dass die Verriegelungsdrähte (8) um eine Verriegelungsschraube (10) auskröpfbar sind.

2. Markraumnagel nach Anspruch 1, **dadurch gekennzeichnet, dass** sich der Querschnitt der Nuten (15;16) an deren Öffnungen in der Mantelfläche (27) des Nagelkörpers (1) verengt, so dass die Verriegelungsdrähte (8) quer zur Längsachse (3) nicht aus den Nuten (15;16) entweichen können.

3. Markraumnagel nach Anspruch 1 oder 2, **dadurch gekennzeichnet, dass** die Nuten (15;16) oder Bohrungen im Bereich des unteren Endes (5) mit einem Innengewinde (20) versehen sind.

4. Markraumnagel nach einem der Ansprüche 1 bis 3, **dadurch gekennzeichnet, dass** dieser zusätzlich Verriegelungsdrähte (8) umfasst.

5. Markraumnagel nach Anspruch 4, **dadurch gekennzeichnet, dass** die Verriegelungsdrähte (8) Gewindespitzen (9) aufweisen, deren Gewinde zu den Innengewinden (20) in den Nuten (15;16) oder Bohrungen korrespondieren und welche parallel zur Längsachse (3) über die Nagelspitze (7) hinaus verschiebbar und in den Knochen einschraubbar sind.

6. Markraumnagel nach einem der Ansprüche 1 bis 5, **dadurch gekennzeichnet, dass** der Schaft (6) einen abgeflachten Querschnitt (12) aufweist, wobei aus der Kreisfläche (24) mit dem Durchmesser D zwei Segmente mit parallelen Sehnen (14), welche symmetrisch zur Längsachse (3) angeordnet sind und einen Abstand x aufweisen, entfernt sind.

7. Marknagel nach Anspruch 6, **dadurch gekennzeichnet, dass** das Verhältnis x/D in einem Bereich von 50% bis 80% liegt.

8. Markraumnagel nach Anspruch 7, **dadurch gekennzeichnet, dass** das Verhältnis x/D in einem Bereich von 60% bis 70% liegt.

9. Markraumnagel nach einem der Ansprüche 6 bis 8, **dadurch gekennzeichnet, dass** die Nuten (15;16) in die peripheren Kreisbogen (13) des Querschnitts (12) eingelassen sind.

10. Markraumnagel nach einem der Ansprüche 1 bis 9, **dadurch gekennzeichnet, dass** das Endteil (2) einen kreiszylindrischen Querschnitt aufweist.

11. Markraumnagel nach einem der Ansprüche 1 bis 10, **dadurch gekennzeichnet, dass** dieser zusätzlich eine Verriegelungsschraube (10) umfasst.

12. Markraumnagel nach einem der Ansprüche 1 bis 11, **dadurch gekennzeichnet, dass** der Nagelkörper (1) aus Titan gefertigt ist.

13. Markraumnagel nach einem der Ansprüche 1 bis 11, **dadurch gekennzeichnet, dass** der Nagelkörper (1) aus rostfreiem Stahl gefertigt ist.

14. Markraumnagel nach einem der Ansprüche 1 bis 11, **dadurch gekennzeichnet, dass** der Nagelkörper (1) aus Kunststoff gefertigt ist.

15. Markraumnagel nach Anspruch 14, **dadurch gekennzeichnet, dass** der Markraumnagel (1) aus dem Kunststoff PEEK gefertigt ist.

## Claims

1. A medullary-space nail for surgical treatment of thin, tubular bones in the human body, comprising:
A) a nail shank (1) having a longitudinal axis (3), an upper end (4) and a lower end (5), where
B) the nail shank (1) comprises a prismatic or cylindrical end part (2) situated at the upper end (4) and fitted with means to connect to assembly or disassembly tools, further a longitudinal stem (6) and a nail tip (7) situated at the lower end (5) and simplifying implantation,
C) the nail shank (1) comprises channels (15, 16) configured parallel and excentric to the longitudinal axis (3) or boreholes receiving locking wires (8), whereby the locking wires (8) can be shifted in the channels (15, 16) or boreholes coaxially with the longitudinal axis (3) and beyond the nail tip (7),
**characterized in that**
D) in the vicinity of the upper end (4), the nail shank (1) comprises a continuous borehole (11) with a central axis (23) running transversely to the longitudinal axis (3) in such manner that a locking screw (10) may be inserted in this continuous borehole (11) and as a result it precludes displacement of the nail shank (1) relative to the longitudinal axis (3) as well as a rotation of the nail shank (1) about this longitudinal axis (3); and
E) the channels (15, 16) comprise a recess (21) in the region of the continuous borehole (11), in such manner that the locking wires (8) may be bent around the locking screw (10).

2. Medullary nail as claimed in claim 1, **characterized in that** the cross-section of the channels (15, 16) is constricted at their openings into the outside surface (27) of the nail shank (1) in such manner that the locking wires (8) cannot slip out of the channels (15, 16) transversely to the longitudinal axis (3).

3. Medullary nail as claimed in either of claims 1 and 2, **characterized in that** the channels (15, 16) or the boreholes are fitted with an inside thread (20) in the region of the lower end (5).

4. Medullary nail as claimed in one of claims 1 through 3, **characterized in that** said nail additionally comprises locking wires (8).

5. Medullary nail as claimed in claim 4, **characterized in that** the locking wires (8) comprise threaded tips (9) of which the threads match the inside threads (20) in the channels (15, 16) or boreholes, said tips being displaceable parallel to the longitudinal axis (3) beyond the nail tip (7) and can be screwed into the bone.

6. Medullary nail as claimed in one of claims 1 through 5, **characterized in that** the stem 6 has an oblate cross-section (12), two segments -- with parallel chords (14) symmetric to the longitudinal axis (3) and mutually apart by a distance x -- being removed from the circular surface (24) having a diameter D.

7. Medullary nail as claimed in claim 6, **characterized in that** the ration x/D is in a range of 50 to 80 %.

8. Medullary nail as claimed in claim 7, **characterized in that** the ratio x/D is in a range of 60 to 70%.

9. Medullary nail as claimed in one of claims 6 through 8, **characterized in that** the channels (15, 16) are fitted into the peripheral arcs of circle (13) of the cross-section (12).

10. Medullary nail as claimed in one of claims 1 through 9, **characterized in that** the end part (2) cross-sectionally is circular-cylindrical.

11. Medullary nail as claimed in one of claims 1 through 10, **characterized in that** in addition it comprises a locking screw (10).

12. Medullary nail as claimed in one of claims 1 through 11, **characterized in that** the nail shank (1) is made of titanium.

13. Medullary nail as claimed in one of claims 1 through 11, **characterized in that** the nail shank (1) is made of stainless steel.

14. Medullary nail as claimed in one of claims 1 through 11, **characterized in that** the nail shank (1) is made of plastic.

15. Medullary nail as claimed in claim 14, **characterized in that** the medullary nail (1) is made of the plastic PEEK.

## Revendications

1. Clou intramédullaire pour le traitement par opération des fractures d'os tubulaires minces du corps humain, qui comprend:
A) un corps de clou (1) doté d'un axe longitudinal (3), d'une extrémité supérieure (4) et d'une extrémité inférieure (5) et dans lequel :
B) le corps de clou (1) comprend une partie d'extrémité (2) prismatique ou cylindrique située à l'extrémité supérieure (4) et dotée de moyens d'accouplement à des instruments de montage ou de démontage, une tige longitudinale (6) et une pointe de clou (7) située à l'extrémité inférieure (5) pour faciliter l'implantation et
C) le corps de clou (1) présente des rainures (15, 16) ou des alésages pour la reprise de fils (8) de verrouillage, qui sont disposés parallèlement et en position décentrée par rapport à l'axe longitudinal (3), de telle sorte que les fils de verrouillage (8) puissent coulisser dans les rainures (15; 16) ou les alésages coaxiale ment par rapport à l'axe longitudinal (3) et au-delà de la pointe (7) du clou,
**caractérisé en ce que**
D) dans la région de l'extrémité supérieure (4), le corps de clou (1) est traversé par un alésage (11) qui s'étend transversalement par rapport à l'axe longitudinal (3) et qui est doté d'un axe central (23) de manière à pouvoir insérer une vis de verrouillage (10) dans cet alésage traversant (11) et ainsi empêcher un déplacement du corps de clou (1) par rapport à l'axe longitudinal (3) ainsi qu'une rotation du corps de clou (1) autour de l'axe longitudinal (3) et **en ce que**
E) les rainures (15; 16) présentent la région de l'alésage traversant (11) une découpe qui permet aux fils (8) de verrouillage d'être coudés autour d'une vis de verrouillage (10).

2. Clou médullaire selon la revendication 1, **caractérisé en ce que** la section transversale des rainures (15; 16) au droit de leur ouverture dans la surface d'enveloppe (27) du corps de clou (1) se rétrécit de telle sorte que les fils de verrouillage (8) ne puissent s'échapper des rainures (15; 16) transversalement par rapport à l'axe longitudinal (3).

3. Clou médullaire selon les revendications 1 ou 2, **caractérisé en ce que** les rainures (15; 16) ou les alésages sont dotés d'un filet intérieur (20) dans la région de l'extrémité inférieure (5).

4. Clou médullaire selon l'une des revendications 1 à 3, **caractérisé en ce que** ce dernier comprend en outre des fils de verrouillage (8).

5. Clou médullaire selon la revendication 4, **caractérisé en ce que** les fils de verrouillage (8) présentent des pointes filetées (9) dont le filet correspond au filet intérieur (20) ménagé dans les rainures (15; 16) ou dans les alésages et qui peuvent coulisser parallèlement à l'axe longitudinal (3) au-delà de la pointe (7) du clou et être vissés dans l'os.

6. Clou médullaire selon l'une des revendications 1 à 5, **caractérisé en ce que** la tige (6) présente une section transversale aplatie (12), deux segments à cordes parallèles (14) qui sont disposés symétriquement par rapport à l'axe longitudinal (3) et qui présentent une distance x étant enlevés de la surface circulaire (24) de diamètre D.

7. Clou médullaire selon la revendication 6, **caractérisé en ce que** le rapport x/D est situé dans la plage de 50 % à 80 %.

8. Clou médullaire selon la revendication 7, **caractérisé en ce que** le rapport x/D est situé dans la plage de 60 % à 70 %.

9. Clou médullaire selon l'une des revendications 6 à 8, **caractérisé en ce que** les rainures (15; 16) sont ménagées dans les arcs de cercle périphériques (13) de la section transversale (12).

10. Clou médullaire selon l'une des revendications 1 à 9, **caractérisé en ce que** la partie d'extrémité (2) présente une section transversale cylindrique circulaire.

11. Clou médullaire selon l'une des revendications 1 à 10, **caractérisé en ce que** ce dernier comprend en outre une vis de verrouillage (10).

12. Clou médullaire selon l'une des revendications 1 à 11, **caractérisé en ce que** le corps de clou (1) est réalisé en titane.

13. Clou médullaire selon l'une des revendications 1 à 11, **caractérisé en ce que** le corps de clou (1) est réalisé en acier inoxydable.

14. Clou médullaire selon l'une des revendications 1 à 11, **caractérisé en ce que** le corps de clou (1) est réalisé en matière synthétique.

15. Clou médullaire selon la revendication 14, **caractérisé en ce que** le clou médullaire (1) est réalisé en la matière synthétique PEEK.
